# EUROPEAN PATENT APPLICATION

(11) **EP 3 117 800 A1**
(43) Date of publication of application: **18.01.2017**
(21) Application number: 16178473.1
(22) Date of filing: 07.07.2016
(51) Int. Cl.: A61F 2/12

(54) **SELF-SUPPORTING PROTHESIS FOR PLASTIC AND RECONSTRUCTIVE SURGERY**

(30) Priority: 15.07.2015 IT UB20152200
(71) Applicant: Solidago AG, 3005 Bern (CH)
(72) Inventor: FENAROLI, Privato, 3005 Bern (CH)
(74) Representative: Gualeni, Nadia

(57) **Abstract**

A self-supporting prosthesis, in particular for plastic and reconstructive surgery, comprising a body (100) provided with a main portion (101) suitable to reshape or fill the area of the body of the patient in which the prosthesis is intended to be implanted. The self-supporting prosthesis is characterised in that the body (100) comprises a fixing portion (120) suitable to allow fixing directly on the muscle tissue of the area of implantation.

## Description

The object of the present invention is a self-supporting prosthesis, in particular for plastic and reconstructive surgery, for example a breast implant.

In addition, this invention also relates to a method of implantation of a self-supporting prostheses for plastic surgery, for example, a breast implant.

As is known, plastic surgeries are among the most performed in the world. In particular, breast augmentation, the purpose of which is to increase the volume of the breast, is one of the most common plastic surgeries.

While the purpose of plastic surgery is to remedy aesthetic defects, reconstructive surgery is instead used to reconstruct parts of the body, for example after their removal as a result of diseases or when these parts have been destroyed by traumatic events. With particular reference to breast reconstructive surgery, the need to protect a woman's physical integrity is by now considered so important as to stimulate plastic surgery to develop increasingly effective reconstruction techniques, capable of repairing the extensive destruction consequent for example to radical mastectomy.

Again with reference to plastic and reconstructive breast surgery, various types of breast implant are known, for example anatomical implants (or tear drop implants), which allow achieving a harmonious and natural breast according to the shape of the rib cage, or round implants, which are very useful when one wants a uniform increase in breast volume without changing its shape.

All known breast implants are constituted by a silicone shell, within which is contained, for example, cohesive silicone gel or a physiological solution of saline water.

However, such known implants have drawbacks, in particular during implantation in the human body. One of the methods used for fixing the implant in position provides for inserting or wrapping the implant itself in an outer shell (a sort of bag) that can be sewn directly to muscle tissue.

The same drawbacks during fixing in position are also found in the field of gluteoplasty with implants, the purpose of which is to reshape or fill, flat and relaxed buttocks. Also in this case, the implants used are made of silicone, stronger than breast implants, and specifically for the buttocks.

In the field of plastic and reconstructive surgery, a need is therefore felt for a self-supporting prosthesis that can be directly fixed in an over the muscle position.

The purpose of this invention is to resolve the problems of the known art, taking into account the needs of the field.

This purpose is achieved by a self-supporting prosthesis according to claim 1. The dependent claims describe preferred or advantageous embodiments of the method and of the prosthesis.

The characteristics and advantages of the self-supporting prosthesis and its method of implantation according to this invention will be apparent from the following description, given by way of non-limiting example, in accordance with the accompanying figures, in which:
- Figures 1 and 2 respectively show a perspective view and a front view of a self-supporting prosthesis according to this invention, in an embodiment variant;
- Figure 3 shows the step of fixing the prosthesis of Figure 1 inside the patient's body;
- Figure 4 shows a front view of a self-supporting prosthesis according to this invention, in a further embodiment variant;
- Figure 5 shows the step of fixing the prosthesis of Figure 4 inside the patient's body;
- Figure 6 shows the placement of a self-supporting breast implant according to this invention, in a positioning mode;

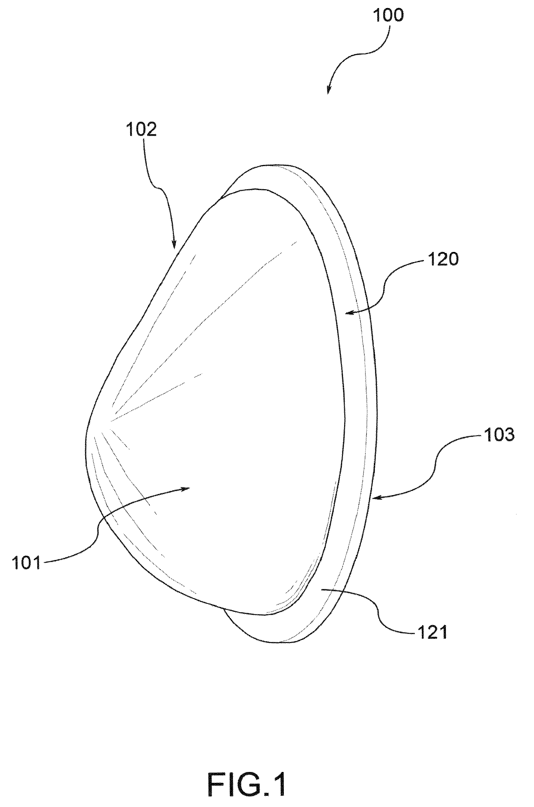

The accompanying figures, and in particular to Figures 1, 2 and 4, show a self-supporting prosthesis. The embodiment examples shown in the figures represent a breast implant. However, the scope of application of the self-supporting prosthesis according to this invention extends to all prostheses for plastic surgery, for example also to implants for gluteoplasty or for other similar surgeries.

The self-supporting prosthesis comprises a body 100 with a main portion 101. The main portion 101 constitutes the prosthesis portion suitable to reshape the area in which the prosthesis will be implanted or to fill the area in which the prosthesis will be implanted (such as the breast or the buttock).

The main portion 101 comprises a back side 103, substantially flat, and a front portion 102, opposite to the back side 103, which defines the shape of the self-supporting prosthesis 100.

The main portion 101 has, for example, a tear drop shape (as in Figure 1) or a round shape.

In an embodiment variant, the main portion 101 contains a filler material, suitable to make the self-supporting prosthesis deformable and natural-looking. The filler material is, for example, cohesive silicone gel, or a physiological saline solution of water, or other flexible and elastic bio-compatible materials of similar, or less density, than that of silicon and of the physiological solution. In this variant, the main portion 101 is a shell, for example silicone, filled with the filler material.

In a further embodiment variant, the main portion 101 is a full body constituted by only one material. This material is suitable to make the self-supporting prosthesis deformable and natural-looking.

In a variant, the material that constitutes the main portion 101 is silicone.

The body 100 is also provided with a fixing portion 120 suitable to allow the fixing of the prosthesis directly on the muscle tissue of the area in which the prosthesis will be implanted. This is to say, the fixing portion 120 constitutes a support for stitching, and in particular for the stitches S, of the prosthesis 100 directly on muscle tissue.

In particular, the self-supporting prosthesis (and thus the body 100) is a single piece. The body 100 is constituted by the main portion 101 and the fixing portion 120, said main portion 101 and fixing portion 120 being integral part of the same body 100 and being made of the same material of the body 100. This is to say, the main portion 101 and the fixing portion 120 are a single body, a single piece.

The fixing portion 120 is at least partially protrudes externally with respect to the main portion 101. Preferably, the fixing portion 120 protrudes with respect to the portion 101 between 5 mm and 20 mm, still more preferably between 10 mm and 15 mm.

Preferably, the fixing portion 120 has a thickness of between 1 mm and 3 mm, still more preferably of 2 mm.

In an embodiment variant, the fixing portion 120 is provided between the back side 103 and the front portion 102 of the main portion 101. In a further embodiment variant, the fixing portion 120 is provided on the front portion 102 of the main portion 101. In a still further embodiment variant, the fixing portion 120 is provided on the back side 103 of the main portion 101.

In an embodiment variant, the fixing portion 120 lies in the same plane of the back side 103, i.e., is coplanar with the back side 103. For example, the fixing portion 120 is an extension of the back side 103, which at least partially protrudes externally with respect to the front portion 102.

In a further embodiment variant, the fixing portion 120 lies on a different plane than that of the back side 103.

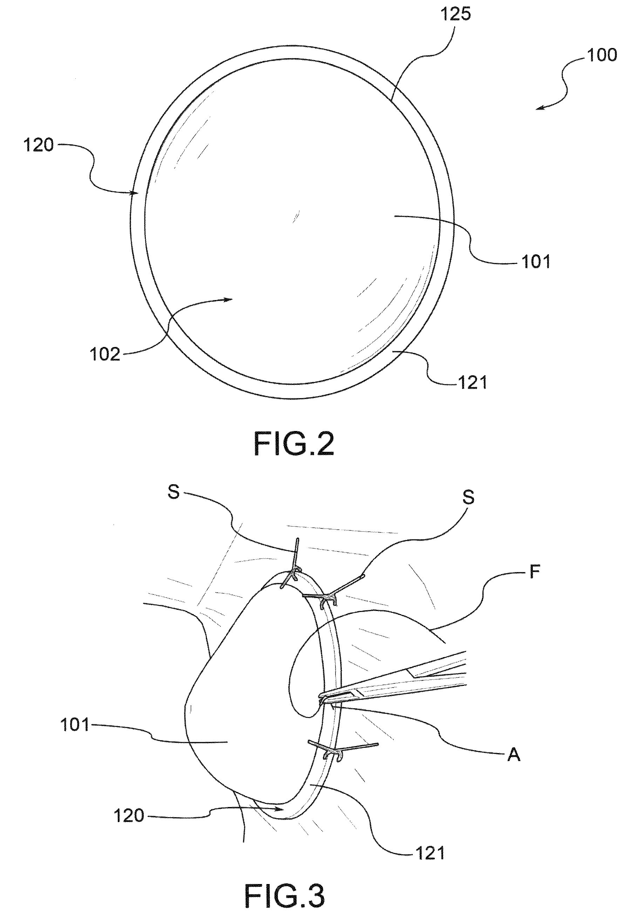

In the variant of Figure 2, the fixing portion 120 is a continuous element disposed around the main portion 101. This constructive choice allows having greater liberty in the step of fixing the self-supporting prostheses, as it is possible to make a suture stitch at any point of the profile of the body 100.

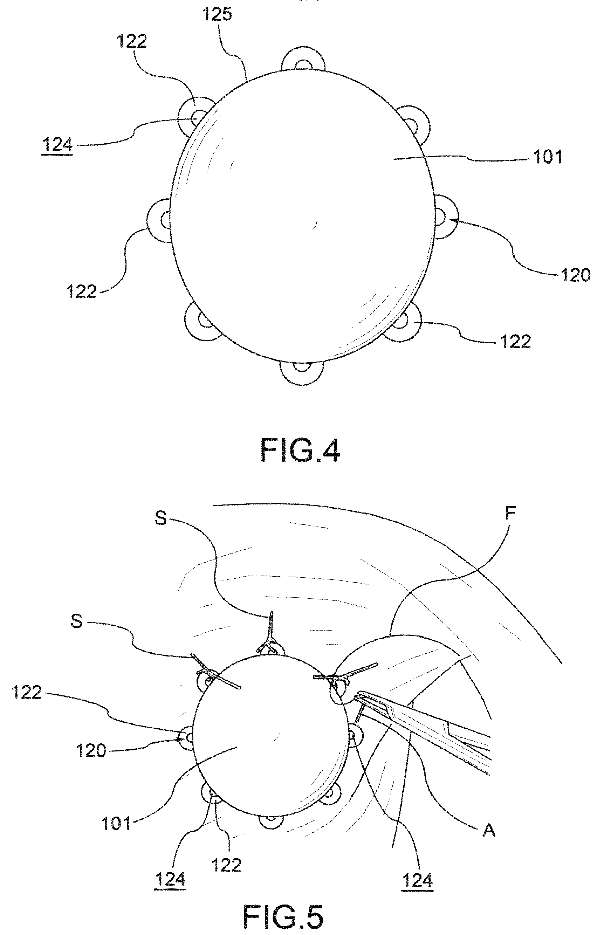

In the variant of Figure 4, the fixing portion 120 is a discontinuous element disposed around the main portion 101.

In the embodiment variant shown in Figures 1 to 3, the fixing portion 120 is suitable to be perforated and passed through by a needle or in general by a pointed element A to allow the stitching of the prosthesis and thus the body 100, directly on the muscle tissue of the area in which the prosthesis will be implanted.

Preferably, the fixing portion 120 has an edge 121, for example annular (for example with a uniform or corrugated profile), preferably provided between the back side 103 and the front portion 102 of the main portion 101.

In an embodiment variant, the edge 121 is continuous.

In a further embodiment variant, the edge 121 is discontinuous.

In the embodiment variant shown in Figures 4 and 5, the fixing portion 120 is a gripping point for suturing, or even better for the suture thread F, to allow the suturing of the prosthesis, and thus the body 100, directly on the muscle tissue of the area in which the prosthesis will be implanted.

Preferably, the fixing portion 120 comprises at least one buttonhole 122 suitable to define a hole 124, or an opening or a passage, for the passage of the suture thread F.

Preferably, the fixing portion 120 comprises a plurality of buttonholes 122. Preferably, the buttonholes 122 are uniformly distributed along the perimeter or profile 125 of the body 100.

In an embodiment variant not shown, the fixing portion 120 is an edge 121 provided with at least one hole 124 for the passage of the suture thread F.

Preferably, the self-supporting prosthesis is provided with a certain surface roughness or corrugation, i.e., that the surface finish of the self-supporting prosthesis is of the type known as textured. This constructive choice allows improving and the surface adhesion of the tissues to the self-supporting prosthesis once it has been implanted in the patient's body.

Preferably, the density range of the composition material of the self-supporting prosthesis and thus the body 100 is comprised between 1.11 g/cm³ and 0.04 g/cm³, preferably from 0.75 g/cm³ and 0.80 g/cm³.

The self-supporting prosthesis is for example a breast implant for breast surgery, an implant for gluteoplasty or a prosthesis for other plastic and reconstructive surgeries.

This invention also relates to a method of implantation of a self-supporting prostheses for plastic and reconstructive surgery.

The implantation method involves the steps of:
- preparing a self-supporting prosthesis comprising a body 100 provided with a main portion 120;
- positioning the self-supporting prosthesis in the patient's body, supported by muscle tissue on which the prosthesis itself will be fixed;
- fixing by suturing the body 100 of the self-supporting prosthesis in such a way as to suture the fixing portion 120 to the patient's muscle tissue.

In particular, the step of positioning the self-supporting prosthesis in the patient's body depends on the type of surgery in progress (for example mastoplasty or gluteoplasty) and the technique used.

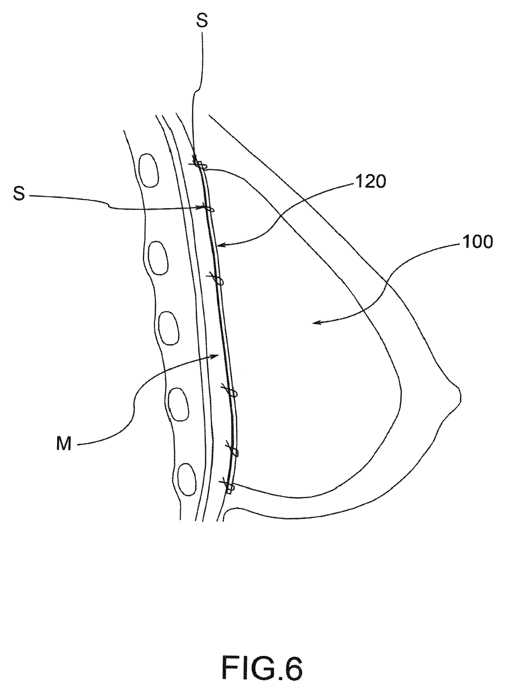

For example, a self-supporting breast implant can be positioned on a sub-glandular surface in the case of plastic surgery, or in place of the removed mammary gland in the case of reconstructive surgery (Figure 6), and the pectoralis major muscle M.

In the case of a self-supporting breast implant, the implantation method involves the steps of:
- preparing a self-supporting prosthesis comprising a body 100 provided with a main portion 120;
- positioning the self-supporting prosthesis resting on the pectoralis major muscle M;
- fixing by suturing the body 100 of the self-supporting prosthesis in such a way as to suture the fixing portion 120 to the patient's pectoralis major muscle M.

For example, a self-supporting prosthesis for gluteoplasty can be accommodated below the muscle fascia or inside the gluteal muscle.

In particular, the step of fixing by suturing the self-supporting prosthesis in such a way as to suture the fixing portion 120 to the patient's muscle tissue depends on the type of self-supporting prosthesis, and in particular the configuration of the fixing portion 120.

For example, in the case of the embodiment variant shown in Figure 3, in which the fixing portion 120 is an edge, the fixing step involves sewing the fixing portion directly to the muscle tissue, i.e., piercing the edge 121 with a pointed element A to which is connected the suture thread F in such a way as to insert the suture thread F in the edge 121 and anchor it to the edge 121 by means of a suture stitch S, for example a knot or a winding.

For example, in the case of the embodiment variant shown in Figure 5, in which the fixing portion 120 comprises at least one buttonhole 122 provided with a hole 124, the fixing step involves inserting the suture thread F directly in the hole 124 and anchoring it to the buttonhole 122 by means of a suture stitch S, for example a knot or a winding.

Innovatively, a self-supporting prosthesis according to this invention is particularly simple and easy to implant in the patient's body, as it does not require additional elements or support structures for fixing in position.

Advantageously, a self-supporting prosthesis according to this invention allows cost savings with respect to a conventional silicone prosthesis as it does not require additional elements or support structures for fixing in position.

Advantageously, a method for implanting a self-supporting prosthesis according to this invention allows obtaining a more effective fixing of the prosthesis directly on muscle tissue.

It is clear that one skilled in the art may make changes to the product described above, all contained within the scope of protection defined by the following claims.

## Claims

1. Self-supporting breast prosthesis, in particular for plastic and reconstructive surgery, comprising a body (100) provided with a main portion (101) suitable to reshape or fill the area of the body of the patient in which the prosthesis is intended to be implanted, and **characterised in that** the body (100) comprises a fixing portion (120) suitable to allow fixing the self-supporting breast prosthesis (100) directly on the body tissue of the implantation area, said main portion (101) and fixing portion (120) being integral part of the same body (100) and being made of the same material of the body (100).

2. Self-supporting breast prosthesis, according to claim 1, wherein the body (10) is a single piece.

3. Self-supporting breast prosthesis according to claim 1 or 2, wherein the density range of the composition material of the body (100) is from 1.11 g/cm³ to 0.04 g/cm³, preferably from 0.75 g/cm³ to 0.80 g/cm³.

4. Self-supporting breast prosthesis, according to any of the preceding claims, wherein the fixing portion (120) is an edge (121) suitable to be perforated or passed through by a pointed element (A) to allow the stitching of the fixing portion (120) directly on the body tissue of the implantation area, said edge (121) being a continuous annular element disposed around the main portion (101).

5. Self-supporting breast prosthesis, according to claim 4, wherein the edge (121) comprises at least one hole (124) for the passage of a suture thread (F).

6. Self-supporting breast prosthesis, according to any of claims from 1 to 3, wherein the fixing portion (120) is a discontinuous element disposed around the main portion (101) and comprises a plurality of buttonholes (122), each buttonhole (122) being provided with a hole (124) for the passage of a suture thread (F), suitable to provide a holding point for the stitching of the fixing portion (120) directly on the body tissue of the implantation area.

7. Self-supporting breast prosthesis, according to any of the preceding claims, wherein the main portion (101) contains a filling material inside it, for example cohesive silicone gel or a physiological solution of saline water.

8. Self-supporting breast prosthesis, according to any of the preceding claims, wherein the fixing portion (120) protrudes externally with respect to the main portion (101) between 5 mm and 20 mm, still more preferably between 10 mm and 15 mm.

9. Self-supporting breast prosthesis, according to any of the preceding claims, wherein the fixing portion (120) has a thickness of between 1 mm and 3 mm, still more preferably of 2 mm.

10. Self-supporting breast prosthesis, according to any of the preceding claims, wherein the fixing portion (120) is coplanar with a back side (103) of the main body (101).
